# EUROPÄISCHE PATENTSCHRIFT

(11) **EP 0 941 228 B1**
(45) Veröffentlichungstag und Bekanntmachung des Hinweises auf die Patenterteilung: **01.08.2001**
(21) Anmeldenummer: 97951215.9
(22) Anmeldetag: 17.11.1997
(51) Int. Cl.: C07D 487/04, C07D 453/04, C08G 65/00

(54) **VERFAHREN ZUR HERSTELLUNG VON MeO-Peg-GESCHÜTZTEN DIHYDROCHININ- ODER DIHYDROCHINIDINDERIVATEN, DIHYDROCHININ- ODER DIHYDROCHINIDINDERIVATE SOWIE VERWENDUNG DERSELBEN**
PROCESS FOR PREPARING MeO-Peg-PROTECTED DIHYDROQUININE OR DIHYDROQUINIDINE DERIVATIVES, DIHYDROQUININE OR DIHYDROQUINIDINE DERIVATIVES AND THEIR USE
PROCEDE DE PREPARATION DE DERIVES DE DIHYDROQUININE OU DE DIHYDROQUINIDINE A MeO-Peg PROTEGE, DERIVES DE DIHYDROQUININE OU DE DIHYDROQUINIDINE ET LEUR UTILISATION

(30) Priorität: 20.11.1996 DE 19647899
(43) Veröffentlichungstag der Anmeldung: 15.09.1999
(73) Patentinhaber: Degussa AG, 40474 Düsseldorf (DE)
(72) Erfinder: BOLM, Carsten, D-52072 Aachen (DE); GERLACH, Arne, D-52062 Aachen (DE); DRAUZ, Karlheinz, D-63579 Freigericht (DE); BOMMARIUS, Andreas, D-60596 Frankfurt (DE)
(86) Internationale Anmeldenummer: EP9706396
(87) Internationale Veröffentlichungsnummer: WO9822466

(56) Entgegenhaltungen:
- US-A- 5 516 929
- HAN; JANDA: "Soluble Polymer-Bound Ligand-Accelerated Catalysis: Asymmetric Dihydroxylation" J. AM. CHEM. SOC., Bd. 118, Nr. 32, 1996, Seiten 7632-33, XP002060030 in der Anmeldung erwähnt
- KOLB ET AL.: "Catalytic Asymmetric Dihydroxylation" CHEM. REV., Bd. 94, Nr. 8, 1994, Seiten 2483-2547, XP002060031 in der Anmeldung erwähnt
- SONG ET AL.: "Polymeric Cinchona alkaloids for the heterogeneous catalytic asymmetric dihydroxylation of olefins: the influence of the polymer backbone polarity on the compatibility between polymer support and reaction medium" TETRAHEDRON ASYMMETRY, Bd. 6, Nr. 11, 1995, Seiten 2687-94, XP002060032

## Beschreibung

Die Erfindung betrifft ein Verfahren zur Herstellung von Dihydrochininderivaten der Formel (I) 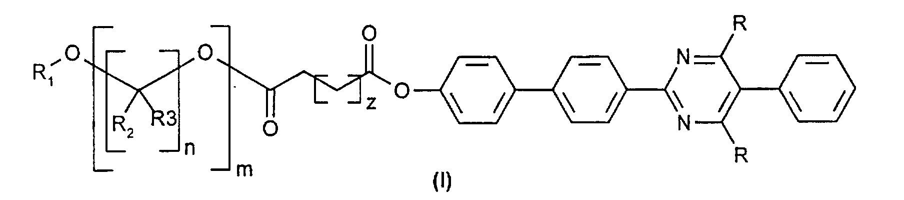
worin m eine ganze Zahl im Bereich von 50 bis 150, n eine ganze Zahl im Bereich von 1 bis 5 und z eine ganze Zahl im Bereich von 0 bis 4 ist,
R₁, R₂ und R₃ unabhängig voneinander gleich oder verschieden, sowie R₂ und R₃ abhängig von n variabel, H, (C₁-C₅) -Alkyl, linear oder verzweigt, (C₃-C₈)-Cycloalkyl, Aryl, Aralkyl, Alkylaryl oder (C₁-C₈)-Alkylalkoxy, linear oder verzweigt, sind
und R ein Rest der Formel (II) oder (III) ist 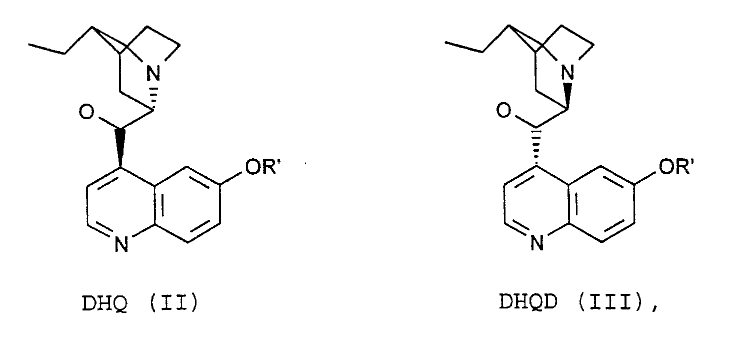
wobei R' für H, (C₁-C₅)-Alkyl, linear oder verzweigt, (C₃-C₈)-Cycloalkyl, Aryl, Aralkyl oder Alkylaryl stehen kann,
sowie zur Herstellung von Dihydrochinidinderivaten der Formel (IV) 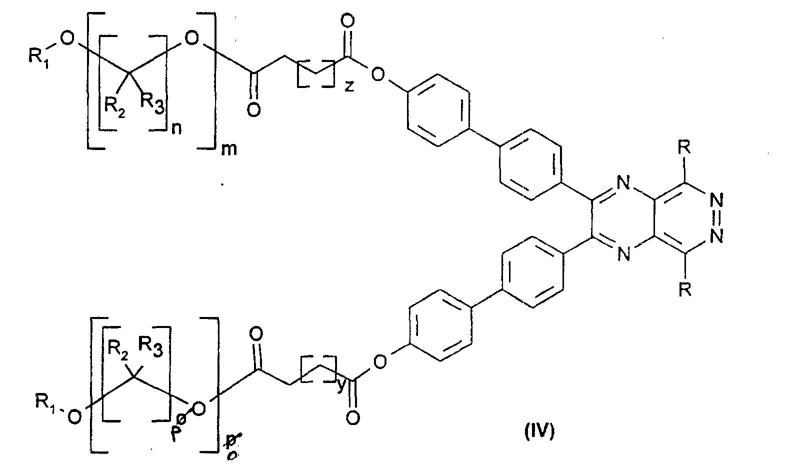
worin m und o unabhängig voneinander gleich oder verschieden ganze Zahlen im Bereich von 50 bis 150, n und p unabhängig voneinander gleich oder verschieden ganze Zahlen im Bereich von 1 bis 5 und z und y unabhängig voneinander gleich oder verschieden ganze Zahlen im Bereich von 0 bis 4 sind und
R, R₁, R₂ und R₃ die bei Formel (I) angegebene Bedeutung besitzen, wobei R₂ und R₃ dabei zusätzlich von o abhängig variabel sind.

Ferner bezieht sich die Erfindung auf nach dem Verfahren der Erfindung erhältliche neue Dihydrochinin- oder Dihydrochinidinderivate der Formeln (I) und (IV) sowie deren Verwendung zur enantioselektiven Dihydroxylierung von Doppelbindungen.

In *Chem. Rev*. **1994**, *94*, 2483 (Sharpless et al.) werden monomere Katalysatorsysteme zur enantioselektiven Dihydroxylierung auf Basis von Dihydrochinin- und Dihydrochinidinderivaten beschrieben. Obwohl die Enantioselektivität der eingesetzten Katalysatoren sehr hoch ist, sind die eingesetzten Liganden insofern nachteilig, als sie sich nicht oder nur schlecht recyclieren lassen und wenn ja, dann nur mit schlechter Ausbeute (Flüssig-flüssig-Extraktionsausbeute deutlich unter 80 %).

In *J*. Am. *Chem. Soc*. **1996**, *118*, 7632-3 (Janda et al.) wird die Herstellung von MeO-Peg-geschütztem Dihydrochinin sowie dessen Anwendung in der enantioselektiven Dihydroxylierung von Doppelbindungen enthaltenden Verbindungen erwähnt. Das dort vorgestellte Katalysatorsystem erreicht bei der enantioselektiven Dihydroxylierung von Standardverbindungen bis zu >30% schlechtere ee-Werte als die ursprünglich von Sharpless et *al*. gefundenen Systeme.

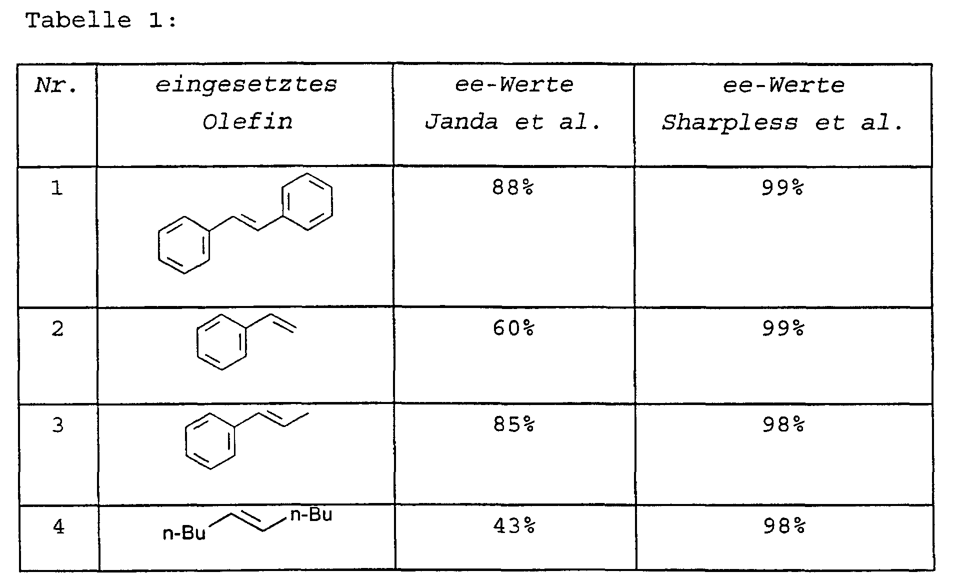

In Tabelle 1 sind die von Janda et *al*. und Sharpless et *al*. erzielten besten Enantioselektivitäten bei der Dihydroxylierung von Standardverbindungen mit ihren Katalysator- oder Ligandensystemen aufgezeigt. Offensichtlich führt die Polymeranbindung der Ligandensysteme bei auf die Einzelsysteme optimierten Reaktionsbedingungen zu drastisch schlechteren ee-Werten.

Aufgabe der Erfindung war es deshalb, ein Verfahren zur Herstellung eines Katalysatorsystems zu entwickeln, welches ähnlich gute ee-Werte wie das original Sharpless-Verfahren bei der Dihydroxylierung liefert und welches in einfacher Weise aus dem Reaktionsmedium abzutrennen ist und so für einen erneuten Reaktionszyklus zur Verfügung steht. Aufgabe der Erfindung war ferner die Angabe neuer Katalysatorsysteme, die zur asymmetrischen Dihydroxylierung von Doppelbindungen dienen können sowie die Art und Weise ihrer Verwendung.

Gelöst wurden diese und weitere nicht näher genannte Aufgaben mit einem Verfahren, das die Merkmale des kennzeichnenden Teils des Anspruchs 1 aufweist. Vorteilhafte Verfahrensmodifikationen werden in den auf Anspruch 1 rückbezogenen Ansprüchen unter Schutz gestellt. In produkttechnischer Hinsicht liefern Ansprüche 8 und 9 Lösungsvorschläge für die der Erfindung zugrundeliegenden Problematik. Die Ansprüche 11-15 offenbaren neuartige Verwendungen der Erfindung.

Indem man Verbindungen der Formel (V) oder (VI) mit Verbindungen der allgemeinen Formel (VII) verestert (vergleiche Schema 1), gelingt es äußerst vorteilhaft Verbindungen der Formeln (I) und (IV) zu erhalten, welche in der enantioselektiven Dihydroxylierung mit nicht ohne weiteres vorhersehbarem Erfolg eingesetzt werden können.

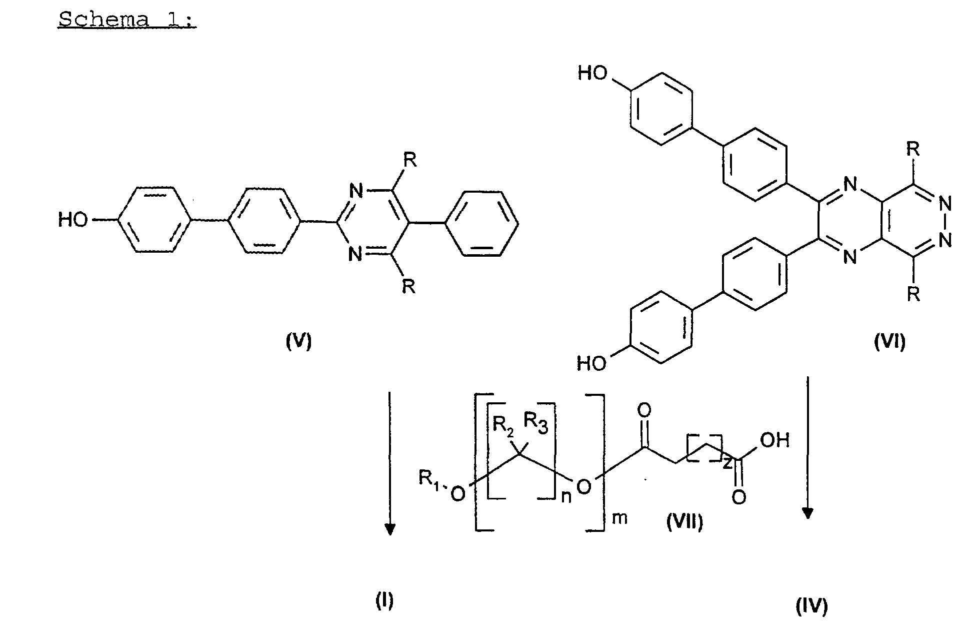

Die Verbindungen der Formeln (V) und (VI) wiederum erhält man, indem man Verbindungen der Formeln (VIII) und (IX) mit dem Aromat (X) in Gegenwart katalytischer Mengen einer Palladium^{±0} -Verbindung umsetzt (vergleiche Schema 2) und anschließend die Silylschutzgruppe mit einem fluoridhaltigen Agenz entfernt. Besonders bevorzugt ist dabei der Einsatz von Tetrabutylammoniumfluorid.

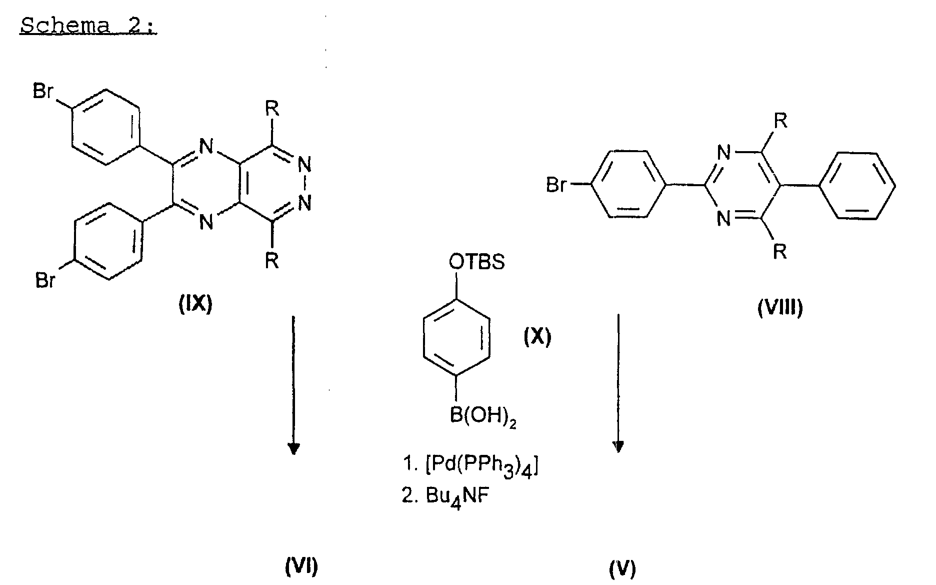

Die eingesetzte Palladiumverbindung besteht vorteilhafterweise aus dem elementaren Metall und einem komplexierenden Liganden aus der Reihe der Triphenylphosphine oder Triphenylphosphite. Ganz besonders bevorzugt ist die Verbindung [Pd(PPh₃)₄].

Die Verbindungen der Formeln VIII und IX kann man auf verschiedene Weisen erhalten.

In einer ersten bevorzugten Ausführungsform kennzeichnet sich das Verfahren der Erfindung dadurch, daß man die Verbindungen der Formeln (VIII) 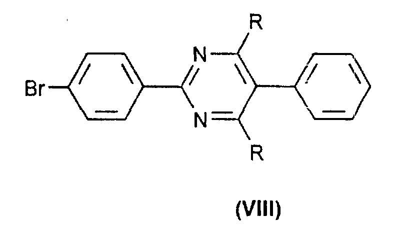 durch Reaktion von Substanzen der Formel. (XII) 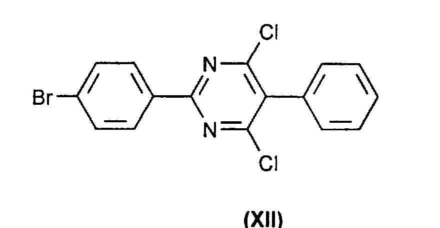 mit DHQ (II) oder DHQD (III) erhält.

In einer zweiten bevorzugten Ausführungsform kennzeichnet sich das Verfahren der Erfindung dadurch, daß man die Verbindung der Formel (IX) 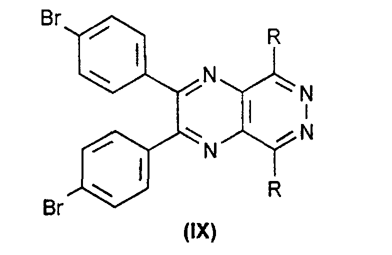 durch Reaktion von Substanzen der Formel (XIII) 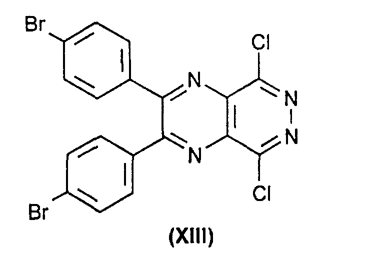 mit DHQ (II) oder DHQD (III) erhält.

Die Substanzen der Formel (XIII) und (XII) können analog literaturbekannter Verfahren (*J*. *Org*. *Chem* **1993**, *58,* 3785) ausgehend von 4-Brombenzonitril oder aus Pyrazin-2,3-dicarbonsäure gemäß z. B. *J*. *Org*. *Chem.* **1995**, *60*, 3940 synthetisiert werden.

Gegenstand der Erfindung sind weiterhin die neuen Ligandensysteme der Formeln (I) und (IV).

Gegenstand der Erfindung ist auch die Verwendung der neuen Ligandensysteme (I) und (IV), die es vorteilhafter Weise in Gegenwart von Oxidationsmitteln wie N-Methylmorpholin-N-oxid, Kaliumhexacyanoferrat und/oder Kaliumosmat in einem Lösungsmittelgemisch gestatten, eine Doppelbindung in sehr hohen Enantiomerenüberschüssen zu dihydroxylieren. Eine bevorzugte erfindungsgemäße Verwendung sieht vor, daß man die Dihydroxylierung in einem Lösungsmittelgemisch aufweisend ein oder mehrere Lösungsmittel der Gruppe, Wasser, Alkoholen wie Methanol, Ethanol, Isopropanol, n-Propanol, n-Butanol, sek.-Butanol, tert.-Butanol, Isobutanol, n-Pentanol, Ethern wie Diethylether, Tetrahydrofuran, Dimethoxyethan, Dioxan, Ketonen wie Aceton, Methylisobutylketon, Ethylketon, Diisopropylketon oder Estern wie Acetessigester oder Essigester sowie halogenierten Alkanen wie Methylenchlorid, Chloroform, Trichlorethylen durchführt. Bevorzugte Lösungsmittelgemische sind u. a. Wasser und tert.-Butanol oder Wasser und Aceton. Besonders zweckmäßig besteht das Lösungsmittelgemisch aus wenigstens zwei der vorgenannten Lösungsmittel. Außerdem können die Katalysatoren (I) und (IV) nach der Dihydroxylierung in besonders einfacher Weise durch Zugabe eines unpolaren organischen Lösungsmittels zum Reaktionsgemisch ausgefällt werden. Zu den hierzu bevorzugt einsetzbaren Lösungsmitteln zählen z. B. Alkane, wie Hexan, Cyclohexan, Methylcyclohexan; Petrolether oder Ether, bevorzugt sind MTBE, Tetrahydrofuran oder Diethylether sowie DME; Ketone, wie Aceton, MIBK oder Ethylmethylketon sowie Diisopropylketon; Ester, wie Essigester oder Acetessigester. Die Temperatur der Dihydroxylierung liegt bei -20° bis +20°C, bevorzugt sind Temperaturen von -10° bis +10°C und ganz besonders bevorzugt sind Temperaturen von +2° bis -2°C. Die Recyclierung der Liganden wird durch Schema 3 veranschaulicht.

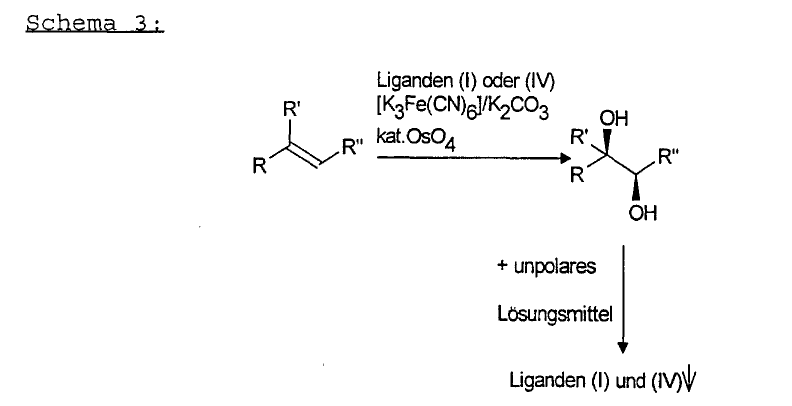

**Tabelle 2:**

| *R,R',R' '* | *Kat.* | *%ee** | *%ee*⁺ |
|---|---|---|---|
| Ph,H,Ph | (IV) | 99% | 99% |
| Ph,H,H | (IV) | 98% | 99% |
| Ph,Me,H | (IV) | 95% | 96% |
| C₈H₁₇, H, H | (I) | 87% | 89% |
| Me₃C,H,H | (I) | 90% | 92% |

| | | | |
|---|---|---|---|
| *: Erfindung ⁺: Sharpless | | | |

Tabelle 2 zeigt die analog Beispiel 1 erhaltenen Enantioselektivitäten der Dihydroxylierung von Standardverbindungen im Vergleich zu den mit dem Katalysator-System von Sharpless et *al*. erreichbaren Werten. Die erfindungsgemäßen ee-Werte liegen nur geringfügig tiefer.

Nach Abtrennen der Liganden vom Reaktionsgemisch durch Ausfällen mit einem unpolaren Lösungsmittel in über 98% Ausbeute können diese in eine neue Dihydroxylierung eingesetzt werden, was äußerst vorteilhaft und dennoch unerwartet war.

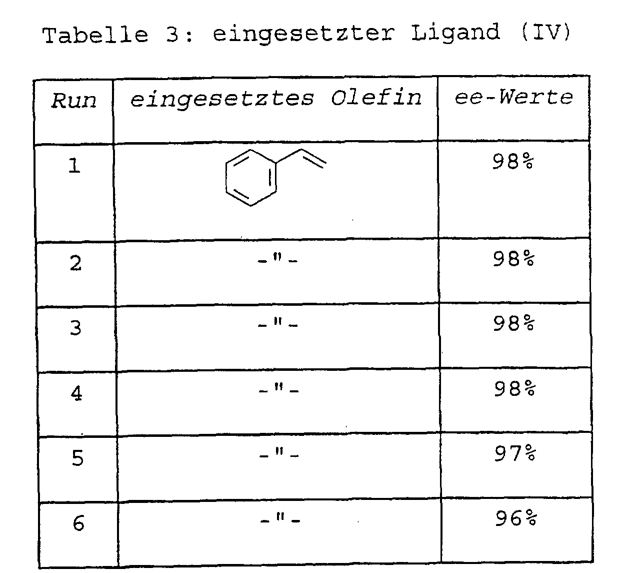

Tabelle 3 zeigt die analog Beispiel 1 erhaltenen Ergebnisse der Dihydroxylierung von Styrol bei sechsmaligem sequentiellen Einsatz des Liganden (IV). Das leichte Absinken der ee-Werte ist auf einen Alkaloidverlust durch geringfügige Esterhydrolyse unter basischen Reaktionsbedingungen zurückzuführen.

Optional kann die Reaktion in geeigneten Anlagen kontinuierlich betrieben werden, indem man mit dem Polymerliganden der Formeln (I) oder (IV) in einem Schlaufenreaktor arbeitet und die Lösung mit der zu hydroxylierenden Verbindung vor der Dihydroxylierung kontaktiert und sie nach Ende der Reaktion durch geeignete Vorrichtungen mit oder ohne vorherigem Ausfällen der Liganden aber unter deren Zurückhalten im Schlaufenreaktor abtrennt.

Es gelingt somit sehr vorteilhaft und in sehr guten Ausbeuten die teuren neuen Ligandensysteme (I) und (IV) in der hoch enantioselektiven Dihydroxylierung einzusetzen und anschließend in einfacher Weise zu recyclieren, was zur wirtschaftlicheren Herstellung von enantioselektiv angereicherten 1,2-Diolen beiträgt.

Die nachfolgenden Beispiele dienen zur eingehenden Erläuterung der Erfindung.

### A: Reaktionsbeispiel

### Beispiel 1:

### Dihydroxylierung von Styrol

Eine Mischung aus 167 mg (15 µmol) (MeOPEG)₂DPP(DHQD)₂, 0.99 g (3 mmol) Kaliumhexacyanoferrat (III), 0.41 g (3 mmol) Kaliumcarbonat und 3.7 mg (10 µmol) Kaliumosmat in 10 ml t-BuOH-Wasser 1:1 wird auf 0 °C mit einem Eisbad abgekühlt. Zu dieser Reaktionslösung tropft man langsam unter starkem Rühren 104 mg (1 mmol) Styrol. Nach 3 h wird die Mischung bei 0 °C vorsichtig mit 1 g Natriumdisulfit versetzt und auf RT aufgewärmt. Es wird mit 10 ml Methylenchlorid verdünnt und von der wässrigen Phase abgetrennt. Aus der organischen Phase wird der Ligand durch langsames Zutropfen von MTBE unter starkem Rühren ausgefällt und mit guter Ausbeute (164 mg, 98 %) zurückgewonnen. Das dihydroxylierte Styrol verbleibt in der organischen Lösung und kann durch Einengen und Chromatographieren an Kieselgel mit MTBE in Ausbeuten von bis zu 127 mg (92 % der Theorie) und 98 % ee isoliert werden.

### B: Herstellung der Liganden

### Beispiel 2:

### Synthese von 4-Brombenzamidinhydrochlorid

Zu einer Lösung von 0.35 g (15 mmol) Natrium in 150 ml Methanol wurden 27.30 g (150 mmol) 4-Brombenzonitril gegeben. Nach 48 h Rühren bei RT wurden 8.00 g (150 mmol) Ammoniumchlorid hinzugefügt, und es wurden weitere 24 h gerührt. Überschüssiges Ammoniumchlorid wurde abfiltriert und mit jeweils 100 ml Methanol und Methylenchlorid gewaschen. Nach Entfernen der Lösungsmittel erhält man einen farblosen Feststoff, der anschließend mit 150 ml Diethylether gewaschen wurde. Die Ausbeute betrug 11.76 g (50 mmol, 33 % der Theorie).

Aus der etherischen Waschlösung konnte man nach Einengen 16.57 g (91 mmol) 4-Brombenzonitril zurückgewinnen.
¹H-NMR (300 MHz, DMSO-d₆): δ 3.17 (s, 4H; NH), 7.80-7.92 (m, 4H; Ar-H)

### Synthese von 2-(4-Bromphenyl)-5-phenyl-4,6-dihydroxypyrimidin

In eine Lösung von 3.45 g (150 mmol) Natrium in 100 ml Methanol wurden nacheinander 10.98 g (47 mmol) 4-Brombenzamidinhydrochlorid und 11.15 g (47 mmol) Phenylmalonsäureethylester eingetragen. Das Gemisch wurde 12 h auf 80 °C erhitzt. Die tiefgelbe Reaktionslösung wurde anschließend von gebildetem Natriumchlorid abfiltriert und das Pyrimidin mit 10 ml 2 molarer Salzsäure als intensivgelber Feststoff ausgefällt. Der Feststoff wurde nacheinander mit Wasser, Ethanol und Diethylether gewaschen. Ausbeute: 12.70 g (37 mmol, 79 % der Theorie) gelber Feststoff. Das Pyrimidin ist in allen getesteten organischen Lösungsmitteln und Wasser unlöslich.
Schmp.: >230 °C
- HRMS (FD) :: berechnet für C₁₆H₁₁BrN₂O₂(M⁺), 342.0009; gefunden 342.0009

### Synthese von 2-(4-Bromphenyl)-5-phenyl-4,6-dichlorpyrimidin

Eine Mischung aus 11 g (32 mmol) 2-(4-Bromphenyl)-5-phenyl-4,6-dihydroxypyrimidin, 120.6 g (0.79 mmol) Phosphorylchlorid und 10.26 g (69 mmol) N,N-Diethylanilin wurde 48 h auf 130 °C erhitzt. Überschüssiges Phosphorylchlorid wurde abdestilliert und der heiße Rückstand unter Rühren zügig auf ein Natriumhydroxid/Eis-Gemisch (27 g/270 g) gegossen. Anschließend wurde dreimal mit jeweils 60 ml Diethylether extrahiert und die vereinigten organischen Phasen nacheinander mit Salzsäure und Wasser neutral gewaschen, über Magnesiumsulfat getrocknet, filtriert und unter vermindertem Druck eingeengt. Umkristallisation des Rückstands aus Essigester/n-Hexan lieferte 9.12 g (24 mmol, 75 % der Theorie) farblose Nadeln.
Schmp.: 133 °C

| | | | |
|---|---|---|---|
| Elementaranalyse: C₁₆H₉Cl₂BrN₂ (380.07 g/mol) : | | | |
| ber. | C 50.56, | H 2.39, | N 7.37; |
| gef. | C 50.54, | H 2.47, | N 7.38 |

### Synthese von 2-(4-Bromphenyl)-5-phenyl-4,6-bis-(dihydrochinidinyl)-pyrimidin

Zu 1.62 g (4.26 mmol) 2-(4-Bromphenyl)-5-phenyl-4,6-dichlorpyrimidin und 2.78 g (8.53 mmol) Dihydrochinidin in 25 ml Toluol wurden 1.8 g (13 mmol) Kaliumcarbonat gegeben und die Mischung wurde 2 h auf 130 °C erhitzt. Anschließend wurden 0.73 g (13 mmol) Kaliumhydroxid zugegeben und das Gemisch 12 h am Wasserabscheider gekocht. Das Toluol wurde abdestilliert, der Rückstand in 15 ml Methylenchlorid aufgenommen und dreimal mit jeweils 10 ml Wasser ausgeschüttelt. Die vereinigten organischen Phasen wurden über Magnesiumsulfat getrocknet, filtriert und im Vakuum eingeengt. Reinigung durch Säulenchromatographie (Kieselgel; Eluent: Chloroform-Ethanol 9:1) ergab 3.06 g (3.2 mmol, 75 % der Theorie) schwach gelben Feststoff.
Schmp.: 125-128 °C

### Synthese von 2-(4-Hydroxybiphenyl)-5-phenyl-4,6-bis(dihydrochinidinyl)-pyrimidin

Zu einer Lösung von 1.44 g (1.5 mmol) 2-(4-Bromphenyl)-5-phenyl-4,6-dichlorpyrimidin und 0.132 g (0.11 mmol) Tetrakis(triphenylphosphin)palladium in 15 ml 2 molarer Natriumcarbonatlösung und 45 ml Toluol wurden langsam 0.475 g (1.88 mmol) 4-(tert.Butyldimethylsilyloxy)-phenylboronsäure in 21 ml Methanol gegeben. Anschließend wurde 24 h zum Rückfluß erhitzt. Nach Abkühlung wurde mit jeweils 50 ml Methylenchlorid und Wasser verdünnt. Die abgetrennte wäßrige Phase wurde noch dreimal mit 10 ml CH₂Cl₂ extrahiert. Die vereinigten organischen Phasen wurden über MgSO₄ getrocknet, abfiltriert und im Vakuum eingeengt. Nach Chromatographieren an Kieselgel mit MTBE (Abtrennung der überschüssigen Boronsäure) erhielt man einen schwach gelben Feststoff. Man löste in 35 ml THF und versetzte bei 0 °C langsam mit 3 ml (3 mmol) Tetrabutylammoniumfluorid (1 molar in THF). Anschließend wurde 15 min bei 0 °C und 45 min bei RT nachgerührt. Nach Quenchen mit 30 ml H₂O wurde das THF im Vakuum entfernt und der Rückstand mit dreimal 10 ml Methylenchlorid extrahiert. Die gesammelten organischen Phasen wurden mit 30 ml H₂O gewaschen, über MgSO₄ getrocknet, filtriert und im Vakuum eingeengt. Reinigung durch Säulenchromatographie (Kieselgel; Eluent: Chloroform-Ethanol 9:1) ergab 1.09 g (1.13 mol, 75 % der Theorie) schwach gelben Feststoff.
Schmp.: 178 °C

### Synthese von [2-(4-Hydroxybiphenyl)-5-phenyl-4,6-bis(dihydrochinidinyl)-pyrimidinl-polyethylenglycolsuccinat

Zu einer Mischung von 1.95 g (2 mmol) 2-(4-Hydroxybiphenyl)-5-phenyl-4,6-bis(dihydrochinidinyl)-pyrimidin, 5.10 g (1 mmol) Bernsteinsäurepolyethylenglycolester (Monoester) und 0.024 g (0.2 mmol) 4-Dimethylaminopyridin (DMAP) wurden 0.454 g (2.8 mmol) Dicyclohexylcarbodiimid (DCC) gegeben. Die Lösung wurde 12 h bei RT gerührt. Der ausgefallene Dicyclohexylharnstoff wurde abfiltriert. Zu dem Filtrat wurde anschließend langsam unter starkem Rühren tert-Butylmethylether (MTBE) getropft und der ausgefallene Niederschlag abfiltriert. Der schwach gelbe Feststoff wurde noch jeweils zweimal in wenig Methylenchlorid aufgenommen und wieder mit MTBE ausgefällt. Man erhält 5.63 g (0.93 mmol, 93 % der Theorie) leicht gelben Feststoff.
Schmp.: 56-59 °C
¹H-NMR (300 MHz, CDCl₃) δ = 0.66 (t, J = 7.2 Hz; 6 H), 0.92 (m, 4H), 1.19-1.83 (m, 10H), 1.95 (m, 2H), 2.48-3.01 (m, 12H), 3.2-3.9 (Polyethylenglycolpeaks), 6.9-7.75 (m, 21H), 8.05 (d, J = 9.04 Hz; 2H), 8.79 (m, 2H)

### Beispiel 3:

### Synthese von 2.3-Bis(4-bromphenyl)-5.8-dihydroxypyrazino-[2,3-d]-pyridazin

Eine Lösung von 7.11 g (50 mmol) 4,5-Diamino-3,6-dihydroxypyridazin und 20 g (54 mmol) 4,4'-Dibrombenzil in 400 ml Eisessig wurde 5 h auf 110 °C erhitzt (nach 10 min bildet sich ein gelber Niederschlag). Es wurde langsam auf RT abgekühlt, der Niederschlag abfiltriert, zweimal mit 50 ml Eisessig und zweimal mit n-Hexan gewaschen. Man erhält 19.03 g (40 mmol, 80 der Theorie) gelben Feststoff.
Schmp.: >240 °C

| | | | |
|---|---|---|---|
| Elementaranalyse: C₁₈H₁₀Br₂N₄O₂ (474.11 g/mol) | | | |
| ber. | C 45.60, | H 2.13, | N 11.82; |
| gef. | C 45.81, | H 2.29, | N 11.85 |

### Synthese von 2,3-Bis(4-bromphenyl)-5,8-dichlorpyrazino-[2,3-d]-pyridazin

Eine Mischung aus 18.00 g (38 mmol) 2,3-Bis(4-bromphenyl)-5,8-dihydroxypyrazino-[2,3-d]-pyridazin und 15.81 g (76 mmol) Phosphorpentachlorid in 200 ml Phosphorylchlorid wurde 90 min auf 120 °C erhitzt. Anschließend wurde das Phosphorylchlorid im Vakuum entfernt und der Rückstand in 50 ml Methylenchlorid aufgenommen. 6 g basisches Aluminiumoxid wurde zugegeben und nach Abfiltrieren über Kieselgel erhielt man 17.67 g (34 mmol, 91 % der Theorie) gelben Feststoff.
Schmp.: 214-218 °C

### Synthese von 2,3-Bis(4-bromphenyl)-5,8-bis-(dihydrochinidyl)-pyrazino-[2,3-d]-pyridazin

Eine Lösung von 0.734 g (2.25 mmol) Dihydrochinidin und 0.581 g (5 mmol) N,N,N',N'-Tetramethylethylendiamin in 16 ml Dimethoxyethan wurde auf -50 °C abgekühlt und langsam mit 1.4 ml (2.25 mmol) N-Butyllithium (15%ige Lösung in n-Hexan) versetzt. Die rote Lösung wurde 15 min gerührt, auf RT aufgewärmt und mit 0.511 g (1 mmol) 2,3-Bis(4-bromphenyl)-5,8-dichlorpyrazino-[2,3-d]-pyridazin versetzt. Anschließend wurde 4 h zum Rückfluß erhitzt, abgekühlt und mit 5 ml Wasser und 25 ml Methylenchlorid verdünnt. Es wurde mit 20 ml gesättigter wäßriger Natriumhydrogencarbonatlösung gewaschen und die wäßrige Phase dreimal mit 20 ml Methylenchlorid extrahiert. Die vereinigten organischen Extrakte wurden über Magnesiumsulfat getrocknet, filtriert und unter vermindertem Druck eingeengt. Reinigung durch Säulenchromatographie (Kieselgel; Eluent: Chloroform-Ethanol 9:1) ergab 0.796 g (0.73 mmol, 73 % der Theorie) gelben Feststoff.
Schmp.: 176-180 °C

### Synthese von 5,8-Bis-(dihydrochinidinyl)-2,3-bis-(4-hydroxybiphenyl)-pyrazino-[2,3-d]-pyridazin

Zu einer Lösung von 19.56 g (18 mmol) 2,3-Bis(4-bromphenyl)-5,8-bis-(dihydrochinidyl)-pyrazino[2,3-d]-pyridazin und 0.5 g (0.433 mmol) Tetrakis(triphenylphosphin)-palladium in 500 ml Toluol und 200 ml 2 molarer Natriumcarbonatlösung wurden langsam 13.56 g (54 mmol) 4-(tert.Butyldimethylsilyloxy)-phenylboronsäure in 800 ml Methanol gegeben. Anschließend wurde 24 h zum Rückfluß erhitzt. Nach Abkühlung wurde die abgetrennte wäßrige Phase dreimal mit 100 ml Methylenchlorid extrahiert, die vereinigten organischen Phasen über MgSO₄ getrocknet, filtriert und im Vakuum eingeengt. Nach Chromatographieren an Kieselgel mit MTBE (Abtrennung der überschüssigen Boronsäure) erhielt man einen gelben Feststoff. Man löste in 200 ml THF und versetzte bei 0 °C langsam mit 72 ml (72 mmol) Tetrabutylammoniumfluorid (1 molar in THF). Anschließend wurde 15 min bei 0 °C und 45 min bei RT nachgerührt. Nach Quenchen mit 350 ml Wasser wurde das THF im Vakuum entfernt und der Rückstand mit 300 ml Ethanol-Methylenchlorid 1:1 extrahiert. Die organische Phase wurde dreimal mit 100 ml Wasser gewaschen, über MgSO₄ getrocknet, filtriert und im Vakuum eingeengt. Reinigung durch Säulenchromatographie (Kieselgel; Eluent: Chloroform-Ethanol 8:2) ergab 13.68 g (12.2 mmol, 68 % der Theorie) gelben Feststoff.
Schmp.: 204-209 °C

### Synthese von [5,8-Bis(dihydrochinidinyl)-2,3-bis-(4-hydroxybiphenyl)-pyrazino-[2,3-d]-pyridazin]-polyethylenglycol-succinat

Zu einer Mischung von 1.34 g (1.2 mmol) 5,8-Bis-(dihydrochinidinyl)-2,3-bis(4-hydroxybiphenyl)-pyrazino-[2,3-d]-pyridazin, 13.26 g (2.6 mmol) Polyethylenglycolsuccinat (Monoester) und 32 mg (0.26 mmol) DMAP wurden 2.7 g (13 mmol) DCC gegeben. Es wurde 48 h bei RT gerührt. Der ausgefallene Dicycloharnstoff wurde abfiltriert. Zu dem Filtrat wurde anschließend langsam unter starkem Rühren MTBE getropft und der ausgefallene Niederschlag abfiltriert. Der gelbe Feststoff wurde noch jeweils zweimal in wenig Methylenchlorid aufgenommen und wieder mit MTBE ausgefällt. Man erhielt 12.24 g (1.10 mmol, 92 % der Theorie) gelben Feststoff.
Schmp.: 55-58 °C

### Synthese von 4-Bromphenol-tert.butyldimethylsilylether

Zu einer Lösung von 31.14 g (180 mmol) 4-Bromphenol und 32.55 g (216 mmol) tert.Butyldimethylsilylchlorid in 600 ml Methylenchlorid wurden 29.4 g (432 mmol) Imidazol gegeben. Anschließend wurde 12 h bei RT gerührt. Nach Quenchen mit 1000 ml Wasser wurde die wäßrige Phase abgetrennt und dreimal mit 200 ml MTBE extrahiert. Die vereinigten organischen Phasen wurden mit gesättigter Natriumchloridlösung gewaschen, über MgSO₄ getrocknet, filtriert und im Vakuum eingeengt. Reinigung durch Säulenchromatographie (Kieselgel; Eluent: MTBE) ergab 50.15 g (17.4 mmol, 97 % der Theorie) klares, farbloses Öl.

| | | |
|---|---|---|
| Elementaranalyse: C₁₂H₁₉BrOSi (287.27 g/mol) | | |
| ber. | C 50.17 | H 6.67 |
| gef. | C 50.24 | H 6.70 |

### Synthese von 4-(tert.Butyldimethylsilyloxy)-phenylboronsäure

Zu einer Suspension von 0.53 g (22 mmol) Magnesiumspäne in 200 ml THF wurden 0.5 ml von 5.75 g (20 mmol) 4-Bromphenol-tert.butyldimethylsilylether und ein Tropfen 1,2-Dibromethan gegeben. Zum Anspringen der Reaktion wurde diese Mischung einmal kurz zum Rückfluß erhitzt. Anschließend wurde langsam der Rest des Arylbromids hinzugetropft. Nach beendeter Zugabe wurde noch 1 h zum Rückfluß erhitzt. Die kupferfarbene Lösung wurde auf RT abgekühlt und langsam zu einer auf -78 °C gekühlten Lösung von 10.39 g (0.1 mol) Trimethylborat in 50 ml THF getropft. Über Nacht ließ man langsam auf RT kommen. Es wurde mit 30 ml Wasser gequencht und das THF im Vakuum entfernt. Der Rückstand wurde dreimal mit MTBE extrahiert. Die vereinigten organischen Phasen wurden über MgSO₄ getrocknet, filtriert und im Vakuum eingeengt. Reinigung durch Säulenchromatographie (Kieselgel; Eluent: MTBE) ergab 4.09 g (16.2 mmol, 81 % der Theorie) farblosen Feststoff.
¹H-NMR (300 MHz, Aceton-d₆) : δ = 0.22 (s, 6H; CH₃), 0.99 (s, 9H; tBu), 6.86 (m, 2H; Ar-H), 7.79 (s, 2H; Ar-H)

| | | |
|---|---|---|
| Elementaranalyse: C₁₂H₂₁BO₃Si (252.19) | | |
| ber. | C 57.15 | H 8.39 |
| gef. | C 60.16 | H 8.44 |

## Patentansprüche

1. Verfahren zur Herstellung von Liganden der Formel (I) oder (IV) 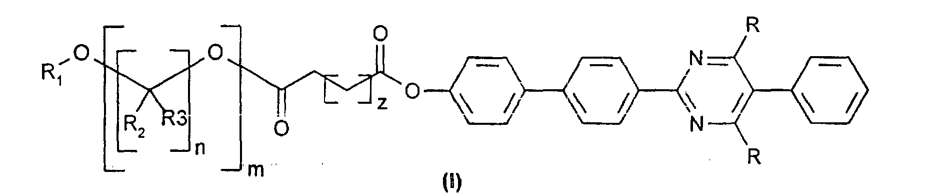
worin m eine ganze Zahl im Bereich von 50 bis 150, n eine ganze Zahl im Bereich von 1 bis 5 und z eine ganze Zahl im Bereich von 0 bis 4 ist,
R₁, R₂ und R₃ unabhängig voneinander gleich oder verschieden, sowie R₂ und R₃ abhängig von n variabel, H, (C₁-C₅)-Alkyl, linear oder verzweigt, (C₃-C₈)-Cycloalkyl, Aryl, Aralkyl, Alkylaryl oder (C₁-C₈)-Alkylalkoxy, linear oder verzweigt, sind
und R ein Rest der Formel (II) oder (III) ist 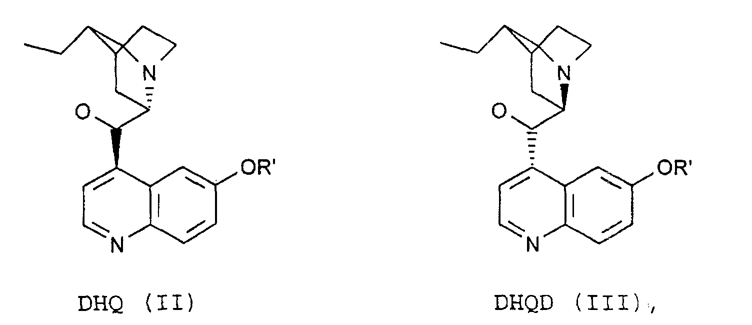
wobei R' für H, (C₁-C₅)-Alkyl, linear oder verzweigt, (C₃-C₈)-Cycloalkyl, Aryl, Aralkyl oder Alkylaryl stehen kann,
ist,
sowie von Dihydrochinidinderivaten der Formel (IV) 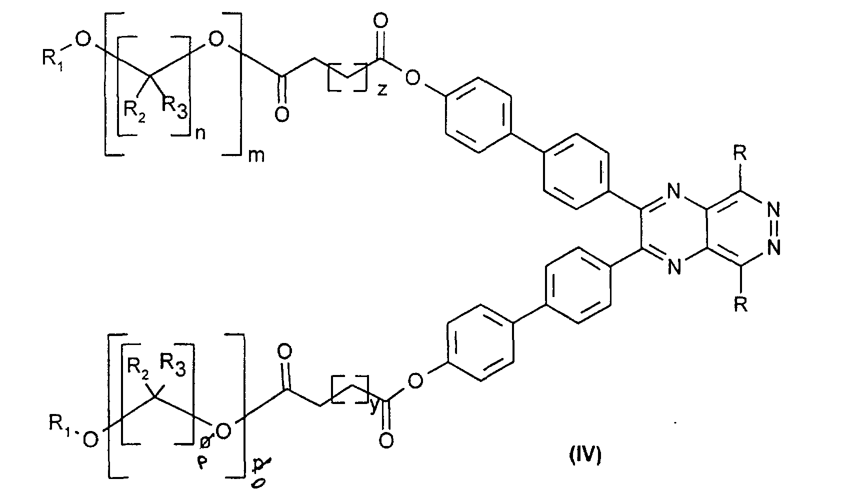
worin m und o unabhängig voneinander gleich oder verschieden ganze Zahlen im Bereich von 50 bis 150, n und p unabhängig voneinander gleich oder verschieden ganze Zahlen im Bereich von 1 bis 5 und z und y unabhängig voneinander gleich oder verschieden ganze Zahlen im Bereich von 0 bis 4 sind und
R, R₁, R₂ und R₃ die bei Formel (I) angegebene Bedeutung besitzen, wobei R₂ und R₃ dabei zusätzlich von o abhängig variabel sind,
**dadurch gekennzeichnet**,
daß Zwischenprodukte der Formel (V) oder (VI), 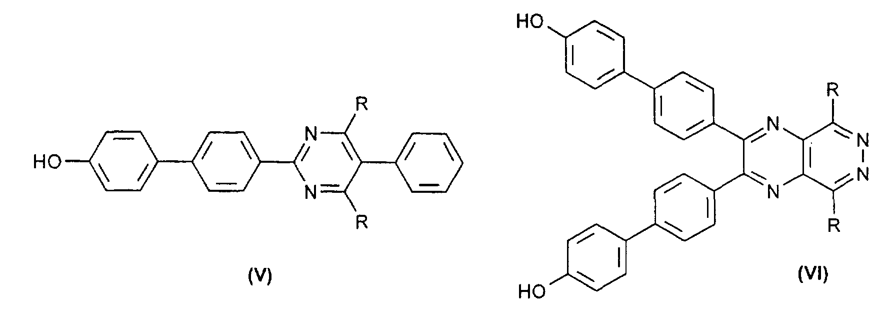 in denen R ein Rest der Formel (II) oder (III) 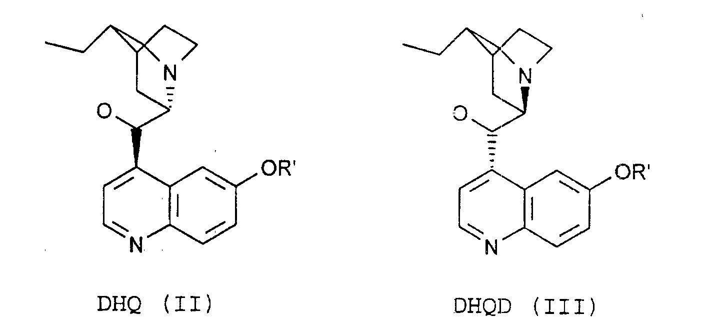
wobei R' für H, (C₁-C₅)-Alkyl, linear oder verzweigt, (C₃-C₈)-Cycloalkyl, Aryl, Aralkyl oder Alkylaryl stehen kann,
ist,
mit Verbindungen der Formel (VII), 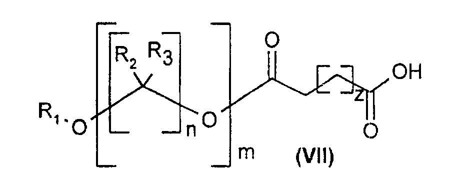
worin m, n und z sowie R₁, R₂ und R₃ die bei (I) und (IV) angegebene Bedeutung besitzen,
verestert werden.

2. Verfahren nach Anspruch 1 dadurch gekennzeichnet, daß man die Zwischenprodukte der Formeln (V) oder (VI) durch Reaktion von Verbindungen der Formel (VIII) und (IX) 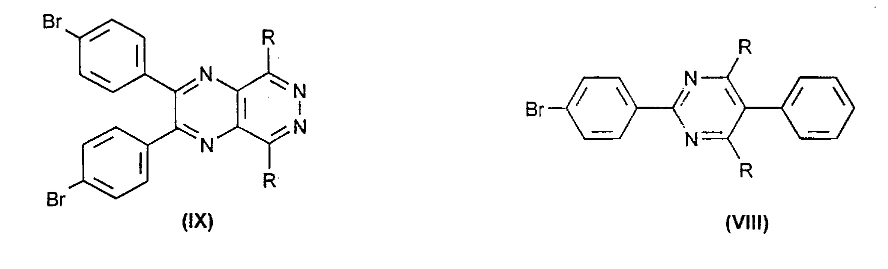 in denen R ein Rest der Formel (II) oder (III) ist, mit einer Verbindung der Formel (X) 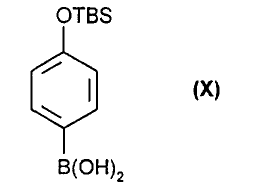 in Gegenwart von katalytischen Mengen an Palladium und anschließender Silylgruppenabspaltung mit fluoridhaltigen Reagenzien erhält.

3. Verfahren nach Anspruch 2,
**dadurch gekennzeichnet**,
daß das katalytisch aktive Palladium die Oxidationsstufe ±0 besitzt und mit Triphenylphosphin- oder Triphenylphosphitliganden komplexiert ist.

4. Verfahren nach Anspruch 3,
**dadurch gekennzeichnet**,
daß als katalytisch aktives Palladium die Verbindung der Formel (XI)
[Pd(PPH₃)₄] (XI)
eingesetzt wird.

5. Verfahren nach Anspruch 2,
**dadurch gekennzeichnet**,
daß man als silylgruppenabspaltende Reagenzien ein oder mehrere Tetraalkylammoniumfluoride verwendet.

6. Verfahren nach Anspruch 5,
**dadurch gekennzeichnet**,
daß man Tetrabutylammoniumfluorid verwendet.

7. Verfahren nach einem oder mehreren der Ansprüche 2-6,
**dadurch gekennzeichnet**,
daß man die Verbindungen der Formeln (VIII) 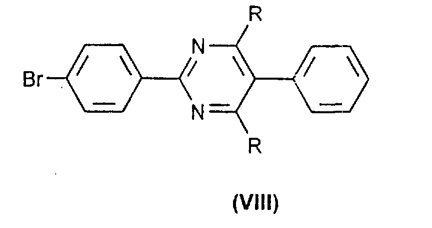 durch Reaktion von Substanzen der Formel (XII) 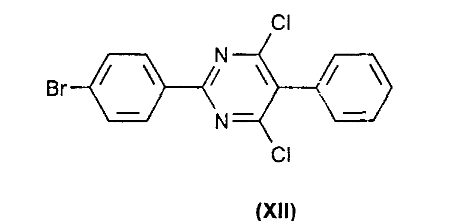 mit DHQ (II) oder DHQD (III) erhält.

8. Verfahren nach einem oder mehreren der Ansprüche 2-6,
**dadurch gekennzeichnet**,
daß man die Verbindung der Formel (IX) 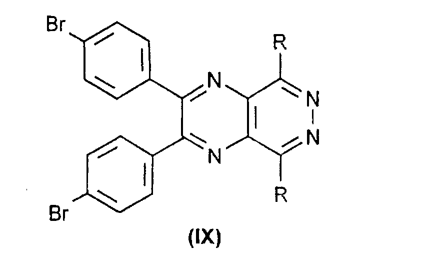 durch Reaktion von Substanzen der Formel (XIII) 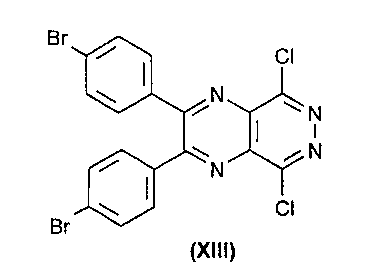 mit DHQ (II) oder DHQD (III) erhält.

9. Liganden der allgemeinen Formel (I) 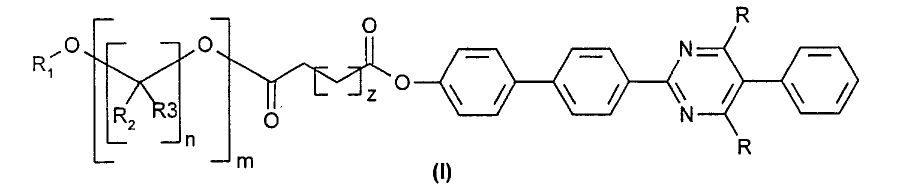
worin m eine ganze Zahl im Bereich von 50 bis 150, n eine ganze Zahl im Bereich von 1 bis 5, und z eine ganze Zahl im Bereich von 0 bis 4 ist,
R₁, R₂ und R₃ unabhängig voneinander gleich oder verschieden, sowie R₂ und R₃ abhängig von n variabel, H, (C₁-C₅)-Alkyl, linear oder verzweigt, (C₃-C₈)-Cycloalkyl, Aryl, Aralkyl, Alkylaryl oder (C₁-C₈)-Alkylalkoxy, linear oder verzweigt, sind
und R ein Rest der Formel (II) 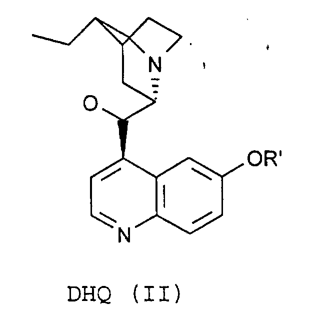 oder ein Rest der Formel (III) 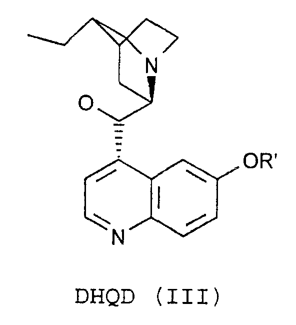 ist,
wobei R' für H, (C₁-C₅)-Alkyl, linear oder verzweigt, (C₃-C₈)-Cycloalkyl, Aryl, Aralkyl oder Alkylaryl stehen kann.

10. Liganden der Formel (IV) 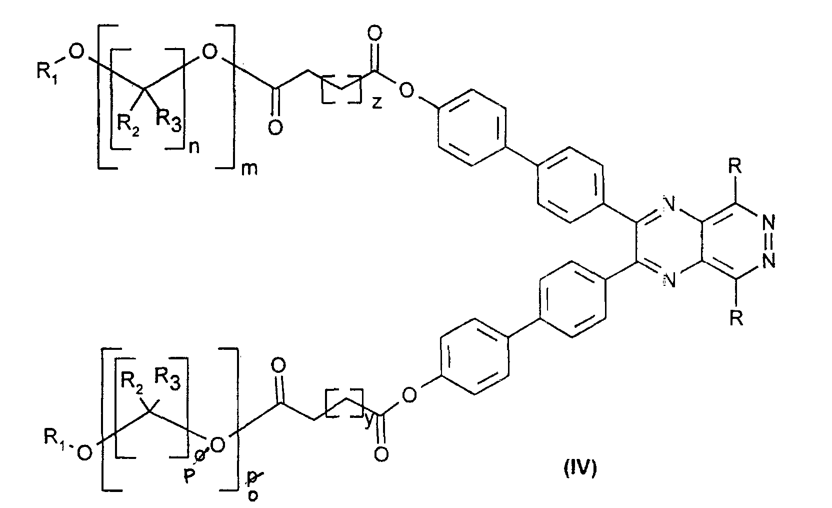
worin m und o unabhängig voneinander gleich oder verschieden ganze Zahlen im Bereich von 50 bis 150, n und p unabhängig voneinander gleich oder verschieden ganze Zahlen im Bereich von 1 bis 5 und z und y unabhängig voneinander gleich oder verschieden ganze Zahlen im Bereich von 0 bis 4 sind und
R, R₁, R₂ und R₃ die bei Formel (I) angegebene Bedeutung besitzen, wobei R₂ und R₃ dabei zusätzlich von o abhängig variabel sind,
und R ein Rest der Formel (II) 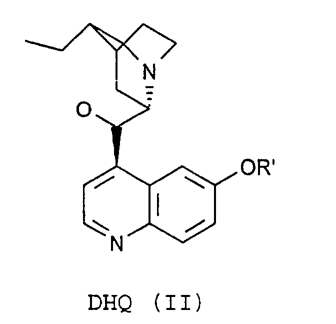 oder ein Rest der Formel (III) 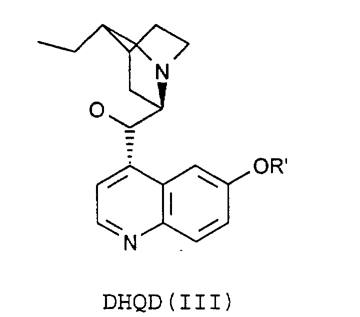 ist,
wobei R' für H, (C₁-C₅)-Alkyl, linear oder verzweigt, (C₃-C₈)-Cycloalkyl, ganz oder teilweise ungesättigt, Aryl, Aralkyl oder Alkylaryl stehen kann.

11. Verwendung der Verbindungen der Formel (I) oder (IV), zur enantioselektiven Dihydroxylierung von Doppelbindungen.

12. Verwendung nach Anspruch 11,
**dadurch gekennzeichnet**,
daß die Verbindungen der Formeln (I) und (IV) nach der Dihydroxylierung ausgefällt, durch Filtration vom Reaktionsgemisch getrennt werden und so erneut in die Reaktion eingesetzt werden können.

13. Verwendung nach einem oder mehreren der Ansprüche 11 und 12,
**dadurch gekennzeichnet**,
daß die Temperatur bei der Dihydroxylierung zwischen -20° und +20°C liegt.

14. Verwendung nach einem oder mehreren der Ansprüche 11 bis 13,
**dadurch gekennzeichnet**,
daß man die Dihydroxylierung in einem Lösungsmittelgemisch aufweisend ein oder mehrere Lösungsmittel der Gruppe, Wasser, Alkoholen wie Methanol, Ethanol, Isopropanol, n-Propanol, n-Butanol, sek.-Butanol, tert.-Butanol, Isobutanol, n-Pentanol, Ethern wie Diethylether, Tetrahydrofuran, Dimethoxyethan, Dioxan, Ketonen wie Aceton, Methylisobutylketon, Ethylketon, Diisopropylketon oder Estern wie Acetessigester oder Essigester sowie halogenierten Alkanen wie Methylenchlorid, Chloroform, Trichlorethylen durchführt.

15. Verwendung nach Anspruch 11,
**dadurch gekennzeichnet**,
daß als Oxidationsmittel Kaliumhexacyanoferrat, N-Methylmorpholinoxid und/oder Kaliumosmat eingesetzt werden.

16. Verwendung nach Anspruch 15,
**dadurch gekennzeichnet**,
daß Kaliumhexacyanoferrat und Kaliumosmat eingesetzt werden.

17. Verwendung nach Anspruch 15,
**dadurch gekennzeichnet**,
daß N-Methylmorpholinoxid und Kaliumosmat eingesetzt werden.

## Claims

1. Process for preparing ligands having the formula (I) or (IV) 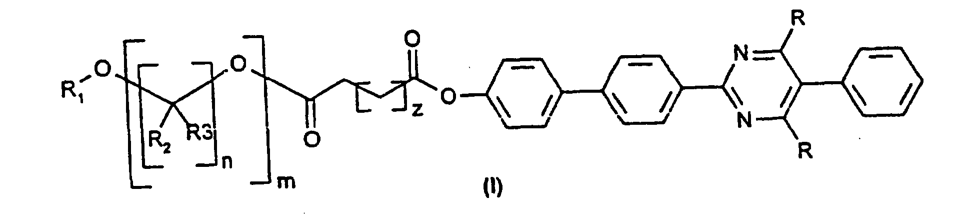
wherein m is a whole number in the range from 50 to 150, n is a whole number in the range from 1 to 5 and z is a whole number in the range from 0 to 4,
R₁, R₂ and R₃ mutually independently identically or differently, and R₂ and R₃ variably depending on n, are H, (C₁-C₅) alkyl, linear or branched, (C₃-C₈) cycloalkyl, aryl, aralkyl, alkylaryl or (C₁-C₈) alkylalkoxy, linear or branched,
and R is a radical having the formula (II) or (III) 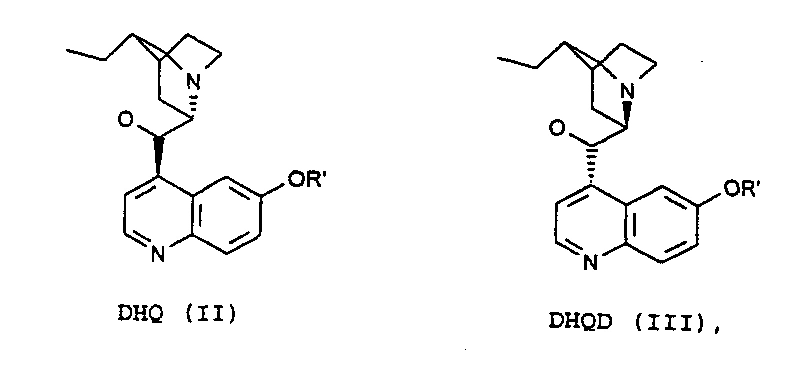
whereby R' can stand for H, (C₁-C₅) alkyl, linear or branched, (C₃-C₈) cycloalkyl, aryl, aralkyl or alkylaryl,
and dihydroquinidine derivatives having the formula (IV) 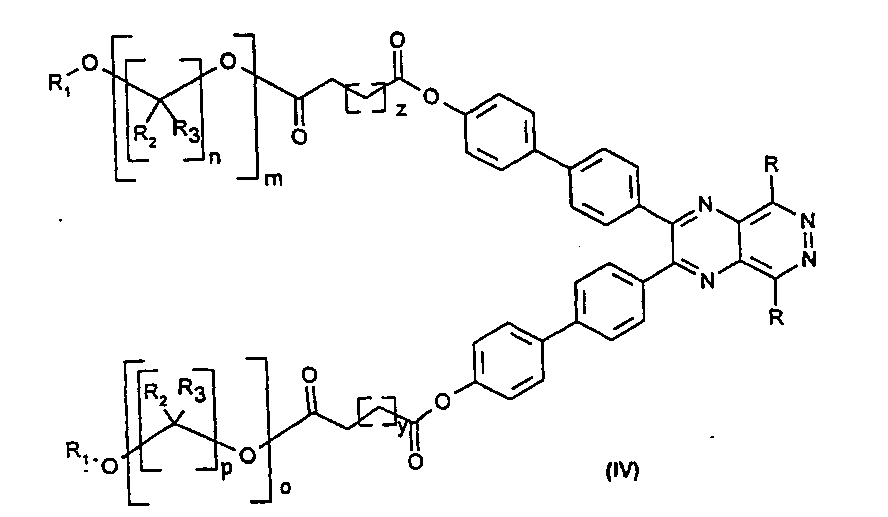
wherein m and o are mutually independently equally or differently whole numbers in the range from 50 to 150, n and p are mutually independently equally or differently whole numbers in the range from 1 to 5 and z and y are mutually independently equally or differently whole numbers in the range from 0 to 4 and
R, R₁, R₂ and R₃ have the meaning given for formula (I), whereby R₂ and R₃ are additionally variable depending on o,
characterised in that
intermediates having the formula (V) or (VI), 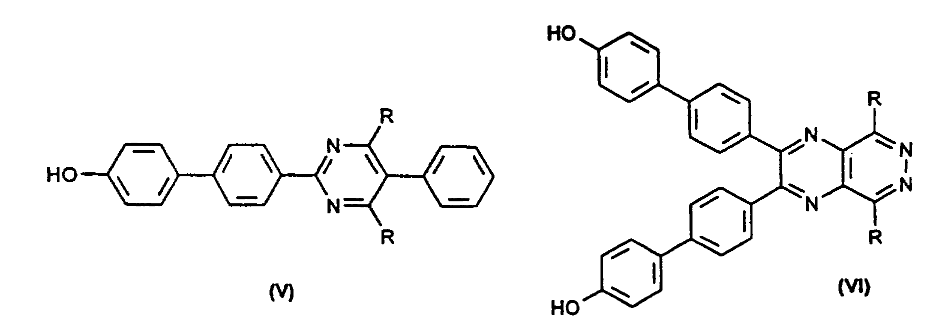 in which R is a radical having the formula (II) or (III) 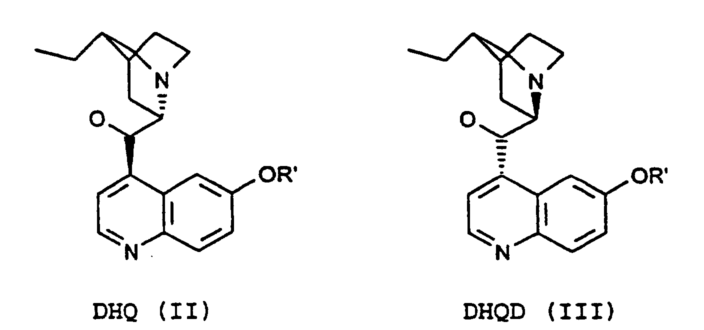
whereby R' can stand for H, (C₁-C₅) alkyl, linear or branched, (C₃-C₈) cycloalkyl, aryl, aralkyl or alkylaryl,
are esterified with compounds having the formula (VII), 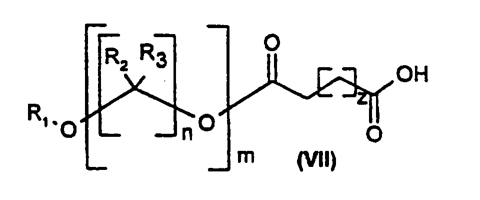
wherein m, n and z and R₁, R₂ and R₃ have the meaning given in (I) and (IV).

2. Process according to claim 1, characterised in that the intermediates having the formulae (V) or (VI) are obtained by reacting compounds having the formula (VIII) and (IX) 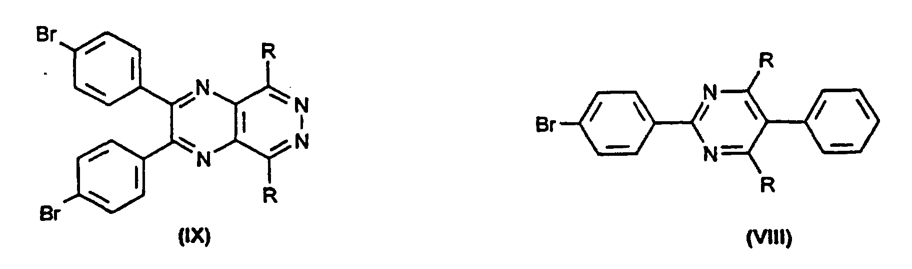 in which R is a radical having the formula (II) or (III),
with a compound having the formula (X) 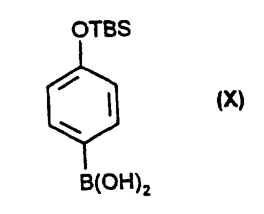 in the presence of catalytic quantities of palladium, followed by elimination of the silyl groups with fluoride-containing reagents.

3. Process according to claim 2,
characterised in that
the catalytically active palladium has the oxidation number ±0 and is complexed with triphenyl phosphine or triphenyl phosphite ligands.

4. Process according to claim 3,
characterised in that
the compound having the formula (XI)
[Pd(PPH₃)₄] (XI)
is used as catalytically active palladium.

5. Process according to claim 2,
characterised in that
one or more tetraalkylammonium fluorides are used as silyl group-eliminating reagents.

6. Process according to claim 5,
characterised in that
tetrabutylammonium fluoride is used.

7. Process according to one or more of claims 2 to 6,
characterised in that
the compounds having the formulae (VIII) 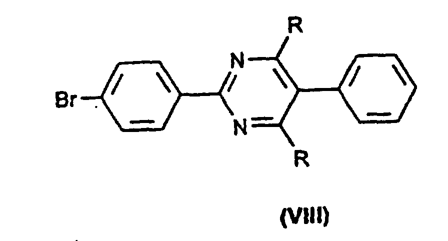 are obtained by reacting substances having the formula (XII) 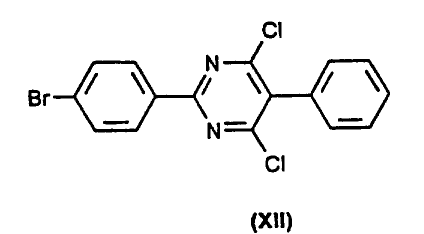 with DHQ (II) or DHQD (III).

8. Process according to one or more of claims 2 to 6,
characterised in that
the compound having the formula (IX) 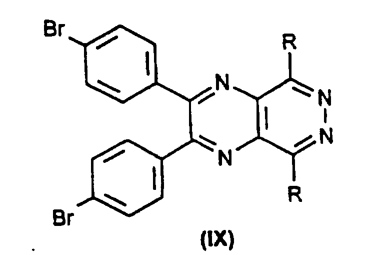 is obtained by reacting substances having the formula (XIII) 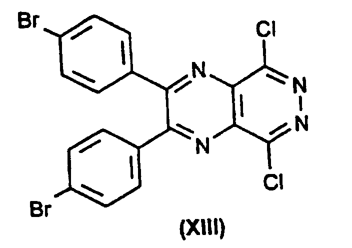 with DHQ (II) or DHQD (III).

9. Ligands having the general formula (I) 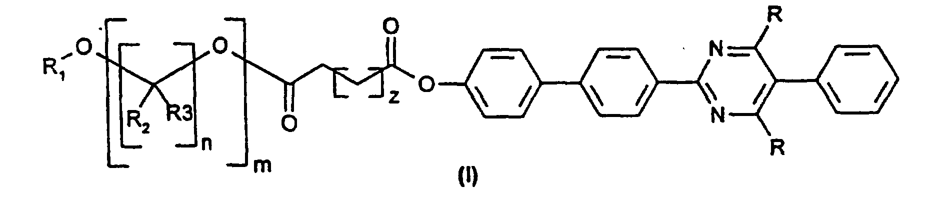
wherein m is a whole number in the range from 50 to 150, n is a whole number in the range from 1 to 5 and z is a whole number in the range from 0 to 4,
R₁, R₂ and R₃ mutually independently identically or differently, and R₂ and R₃ variably depending on n, are H, (C₁-C₅) alkyl, linear or branched, (C₃-C₈) cycloalkyl, aryl, aralkyl, alkylaryl or (C₁-C₈) alkylalkoxy, linear or branched,
and R is a radical having the formula (II) 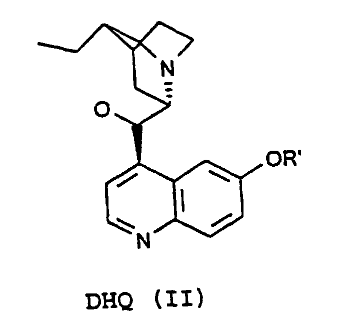 or a radical having the formula (III) 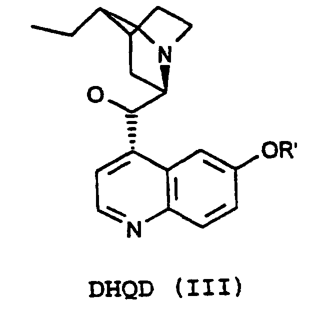
whereby R' can stand for H, (C₁-C₅) alkyl, linear or branched, (C₃-C₈) cycloalkyl, aryl, aralkyl or alkylaryl.

10. Ligands having the formula (IV) 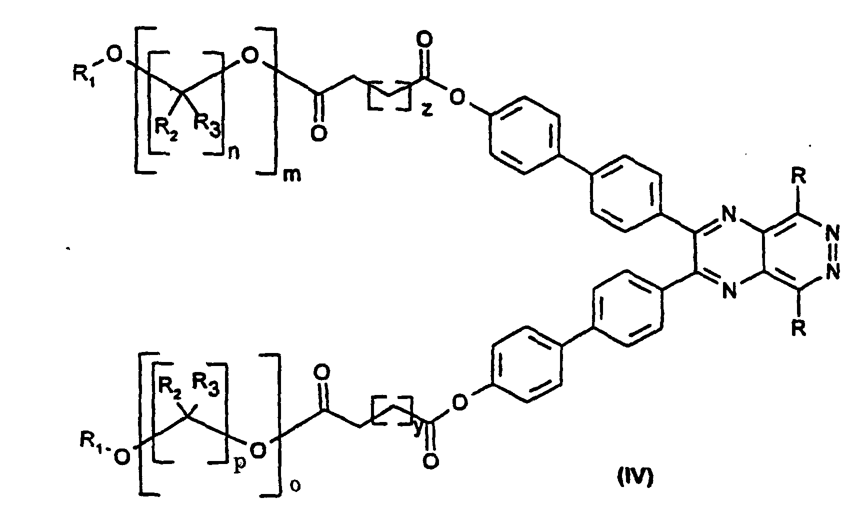
wherein m and o are mutually independently equally or differently whole numbers in the range from 50 to 150, n and p are mutually independently equally or differently whole numbers in the range from 1 to 5 and z and y are mutually independently equally or differently whole numbers in the range from 0 to 4 and
R, R₁, R₂ and R₃ have the meaning given for formula (I), whereby R₂ and R₃ are additionally variable depending on o,
and R is a radical having the formula (II) 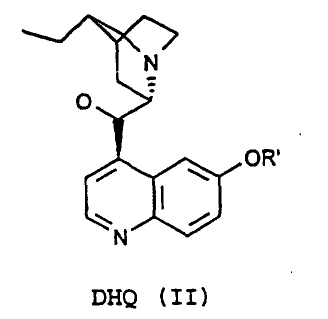 or a radical having the formula (III) 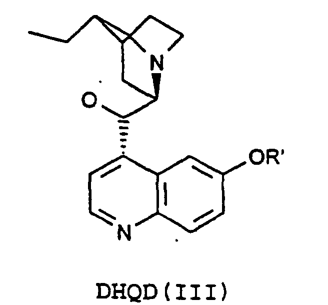
whereby R' can stand for H, (C₁-C₅) alkyl, linear or branched, (C₃-C₈) cycloalkyl, wholly or partially unsaturated, aryl, aralkyl or alkylaryl.

11. Use of the compounds having formula (I) or (IV) in the enantioselective dihydroxylation of double bonds.

12. Use according to claim 11,
characterised in that
the compounds having the formulae (I) and (IV) can be precipitated after dihydroxylation, separated from the reaction mixture by filtration and thereby reused in the reaction.

13. Use according to one or more of claims 11 and 12,
characterised in that the temperature during dihydroxylation is between -20° and +20°C.

14. Use according to one or more of claims 11 to 13,
characterised in that
the dihydroxylation is performed in a solvent mixture displaying one or more solvents from the group of water, alcohols such as methanol, ethanol, isopropanol, n-propanol, n-butanol, sec.-butanol, tert.-butanol, isobutanol, n-pentanol, ethers such as diethyl ether, tetrahydrofuran, dimethoxyethane, dioxan, ketones such as acetone, methylisobutyl ketone, ethyl ketone, diisopropyl ketone or esters such as acetoacetic ester or acetic ester as well as halogenated alkanes such as methylene chloride, chloroform, trichloroethylene.

15. Use according to claim 11,
characterised in that
potassium hexacyanoferrate, N-methylmorpholine oxide and/or potassium osmate are used as oxidising agent.

16. Use according to claim 15,
characterised in that
potassium hexacyanoferrate and potassium osmate are used.

17. Use according to claim 15,
characterised in that
N-methylmorpholine oxide and potassium osmate are used.

## Revendications

1. Procédé de production de ligands de formule (I) ou (IV) 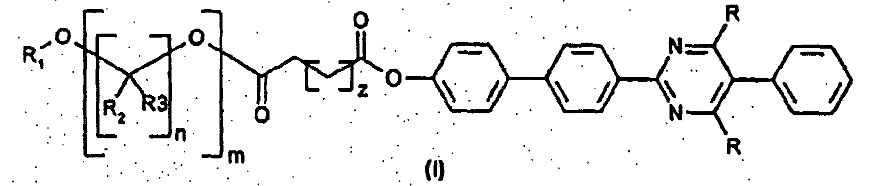
dans lesquels m représente un nombre entier allant de 50 à 150, n un nombre entier allant de 1 à 5 et z un nombre entier allant de 0 à 4, R₁. R₂ ct R₃ représentent indépendamment l'un de l'autre des radicaux identiques ou différents. et R₂ et R₃, dépendant de n de façon variable, sont H, un alkyle en C₁ à C₅, linéaire ou ramifié, un cycloalkyle en C₃ à C₈, un aryle, un aralkyle, un alkylaryle ou un alkyle en C₁ à C₈-alcoxy. linéaire ou ramifié, et R représente un radical de formule (II) ou (III) 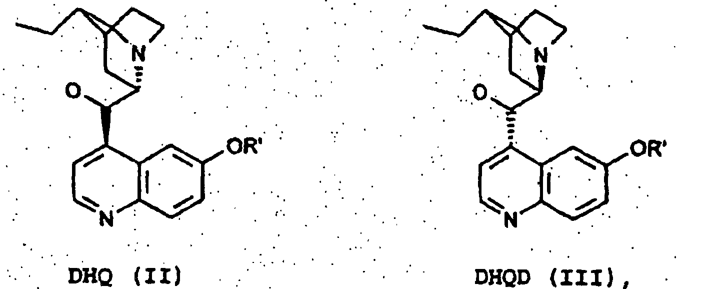
dans laquelle R' peut représenter H, un alkyle en C₁ à C₅, linéaire ou ramifié, un cycloalkyle en C₃ à C₈, un aryle, un aralkyle ou un alkylaryle, ainsi que de dérivés de dihydroquinines de formule (IV) 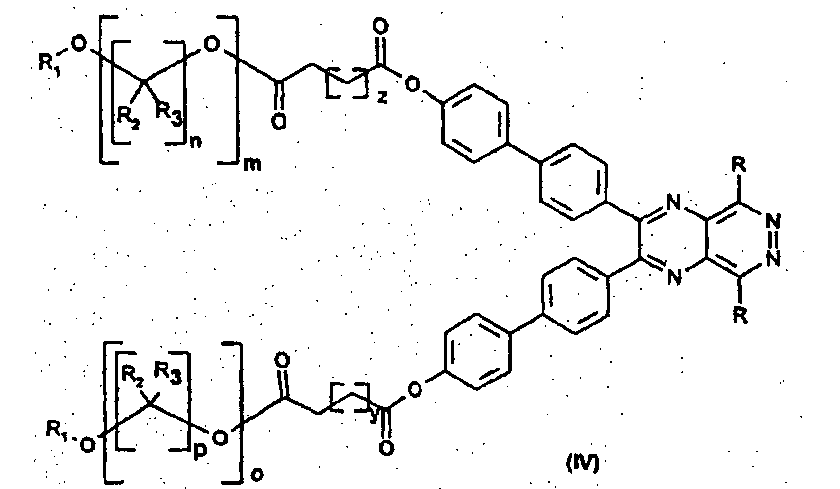
dans laquelle m et o représentent indépendamment l'un de l'autre des nombres entiers identiques ou différents allant de 50 à 150, n et p représentent indépendamment l'un de l'autre des nombres entiers identiques ou différents allant de 1 à 5, et z et y représentent indépendamment l'un de l'autre des nombres entiers identiques ou différents allant de 0 à 4, et R, R₁, R₂ et R₃ ont la signification donnée pour la formule (I), R₂ et R₃ y étant en outre variables d'une manière dépendant de o,
caractérisé en ce qu'
on estérifie des produits intermédiaires de formule (V) ou (VI). 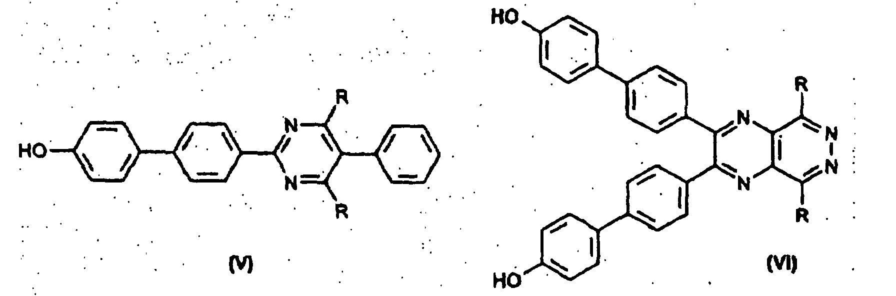
dans laquelle R représente un radical de formule (II) ou (III) 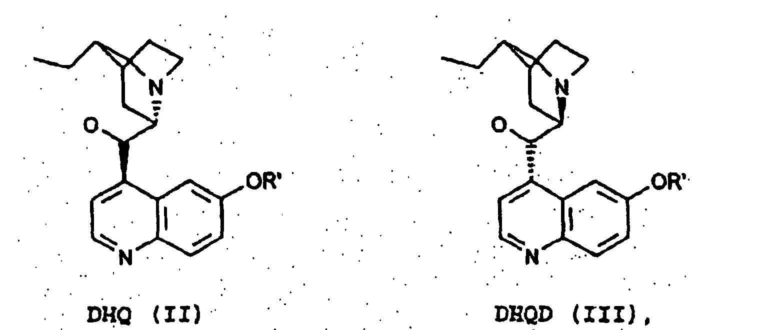
dans lesquelles R' représente H, un alkyle en C₁ à C₅, linéaire ou ramifié, un cycloalkyle en C₃ à C₈. un aryle, un aralkyle ou un alkylaryle. avec des composés de formule (VII). 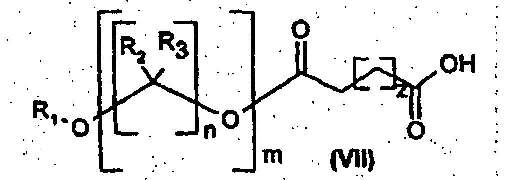
dans laquelle m, n et z ainsi que R₁. R₂ et R₃ ont la signification indiquée pour (I) et (IV).

2. Procédé selon la revendication 1.
caractérisé en ce qu'
on obtient les produits intermédiaires de formules (V) ou (VI) par réaction de composés (VIII) et (IX) 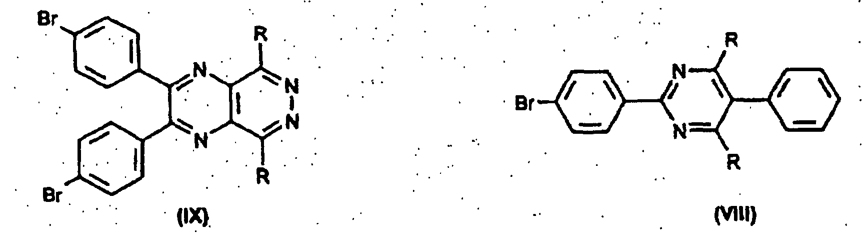 dans lesquelles R est un radical de formule (II) ou (III),
avec un composé de formule (X) 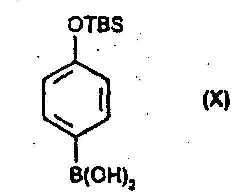 en présence de quantités catalytiques de palladium puis par séparation de groupes silyles avec des réactifs fluorés.

3. Procédé selon la revendication 2,
caractérisé en ce que
le palladium catalytiquement actif possède le degré d'oxydation ± 0 et est complexé avec des ligands de triphényl phosphine ou de triphényl phosphite.

4. Procédé selon la revendication 3.
caractérisé en ce qu'
on utilise comme palladium catalytiquement actif le composé de formule (XI)
[Pd(PPH₃)₄] (XI)

5. Procédé selon la revendication 2.
caractérisé en ce qu'
on utilise comme réactifs donneurs de groupes silyle un ou plusieurs fluorures de tétraalkyl ammonium.

6. Procédé selon la revendication 5.
caractérisé en ce qu'
on utilise du fluorure de tétrabutyl ammonium.

7. Procédé selon une ou plusieurs des revendications 2 à 6,
caractérisé en ce qu'
on obtient les composés de formule (VIII) 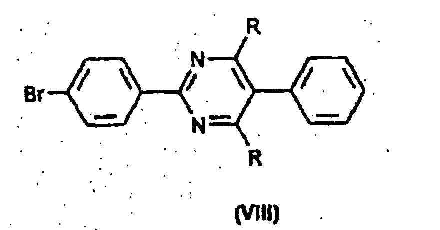 par réaction de substances de formule (XII) 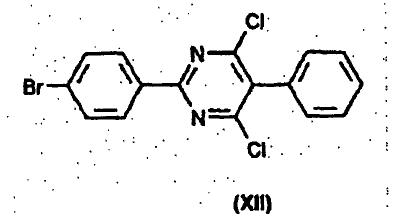 avec DHQ (II) ou DHQD (III).

8. Procédé selon une ou plusieurs des revendications 2 à 6,
caractérisé en ce qu'
on obtient le composé de formule (IX) 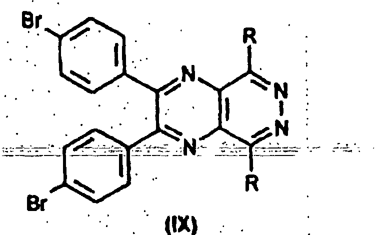 par réaction de substances de formule (XIII) 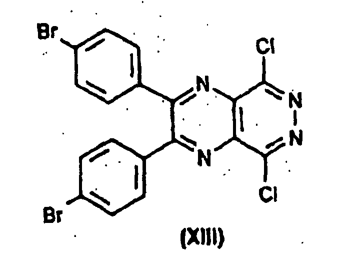 avec DHQ (II) ou DHQD (III).

9. Ligands de formule générale (I) 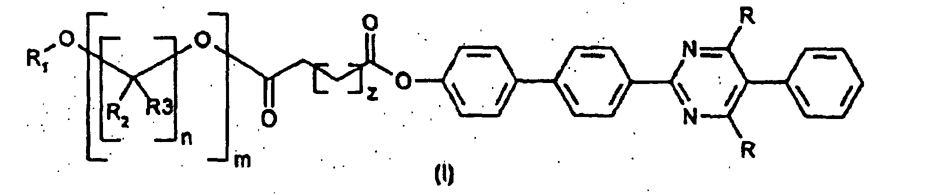
dans laquelle m représente un nombre entier allant de 50 à 150, n représente un nombre entier allant de 1 à 5 et z un nombre entier allant de 0 à 4,
R₁, R₂ et R₃ représentent indépendamment l'un de l'autre des radicaux identiques ou différents, et R₂ et R₃. dépendant de n de façon variable, sont H, un alkyle en C₁ à C₅. linéaire ou ramifié, un cycloalkyle en C₃ à C₈, un aryle, un aralkyle, un alkylaryle ou un alkyle en C₁ à C₈-alcoxy, linéaire ou ramifié, et
R représente un radical de formule (II) 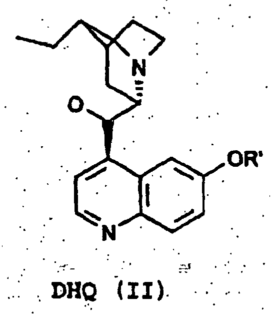 ou un radical de formule (III) 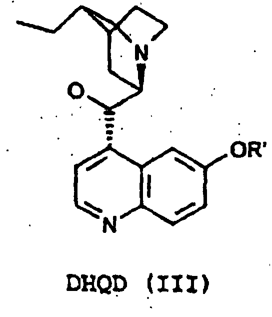
dans laquelle R' peut représenter H, un alkyle en C₁ à C₅, linéaire ou ramifié, un cycloalkyle en C₃ à C₈, un aryle, un aralkyle ou un alkylaryle.

10. Ligands de formule (IV) 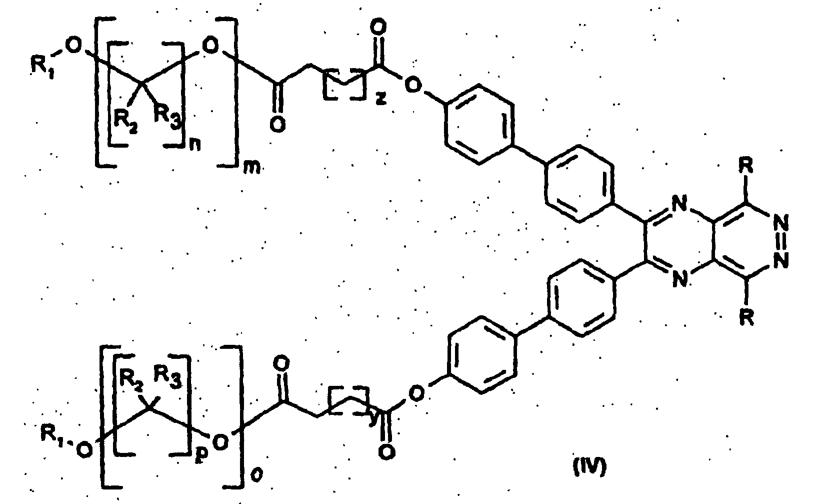
dans laquelle m et o représentent indépendamment l'un de l'autre des nombres entiers identiques ou différents allant de 50 à 150, n et p représentent indépendamment l'un de l'autre des nombres entiers identiques ou dift'érents allant de 1 à 5, et z et y représentent indépendamment l'un de l'autre des nombres entiers identiques ou différents allant de 0 à 4, et
R. R₁, R₂ et R₃ ont la signification donnée pour la formule (I), R₂ et R₃ y étant en outre variables d'une manière dépendante de o,
et R représente un radical de formule (II) 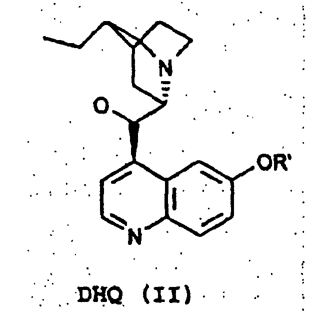 ou un radical de formule (III) 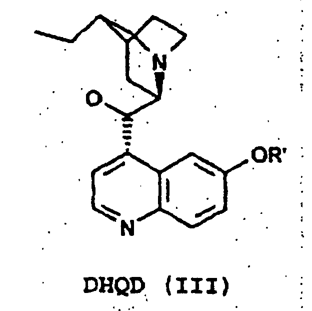
dans lesquelles R' peut représenter H, un alkyle en C₁ à C₅, linéaire ou ramifié, un cycloalkyle en C₃ à C₈, entièrement ou partiellement Saturé, un aryle, un aralkyle ou un alkylaryle.

11. Utilisation des composés de formule (I) ou (IV) pour la dihydroxylation énantioséleclive de doubles liaisons.

12. Utilisation selon la revendication 11,
caractérisée en ce qu'
on précipite les composés de formules (I) et (IV) après la dihydroxylation, on les sépare du mélange réactionnel par filtration, et on peut ainsi les réutiliser dans la réaction.

13. Utilisation selon une ou plusieurs des revendications 11 et 12,
caractérisée en ce que
la température lors de la dihydroxylation s'établit entre -20° et +20°C.

14. Utilisation selon une ou plusieurs des revendications 11 à 13.
caractérisée en ce qu'
on conduit la dihydroxylation dans un mélange de solvants présentant un ou plusieurs solvants du groupe constitué par l'eau, les alcools comme le méthanol, l'éthanol. l'isopropanol, le n-propanol, le n-butanol, le sec.-butanol, le tert.-butanol, l'isobutanol, le n-pentanol, des éthers comme l'éther diéthylique le tétrahydrofuranne, le diméthoxyéthane, le dioxanne, des cétones comme l'acétone, la méthylisobutylcétone, l'éthyl cétone, la dilsopropylcétone ou des esters comme l'acétoacétate d'éthyle ou l'acétate d'éthyle ainsi que des alcanes halogénés comme le chlorure de méthylène, le chloroforme, le trichloroéthylène.

15. Utilisation selon la revendication 11,
caractérisée en ce qu'
on utilise comme oxydant l'hexacyanoferrate de potassium, l'oxyde de N-méthyl morpholine et/ou l'osmate de potassium.

16. Utilisation selon la revendication 15,
caractérisée en ce qu'
on utilise l'hexacyanoferrate de potassium et l'osmate de potassium.

17. Utilisation selon la revendication 15,
caractérisée en ce qu'
on utilise l'oxyde de N-méthyl morpholine et l'osmate de potassium.
